# EUROPEAN PATENT APPLICATION

(11) **EP 4 647 874 A1**
(43) Date of publication of application: **12.11.2025**
(21) Application number: 24175209.6
(22) Date of filing: 10.05.2024
(51) Int. Cl.: G06F 3/01, A63F 13/285, G06F 1/16, G06F 3/03, G06F 3/038

(54) **SYSTEM AND METHOD FOR MODIFYING THE TACTILE STIMULUS WHEN TOUCHING AN OBJECT**

(71) Applicant: Max-Planck-Gesellschaft zur Förderung der Wissenschaften e.V., 80539 München (DE)
(72) Inventor: Strohmeier, Paul, 66123 Saarbrücken (DE); Sabnis, Nihar, 66123 Saarbrücken (DE); Vega, Gabriela, 66123 Saarbrücken (DE); Martinez-Missier, Valentin, 66123 Saarbrücken (DE)
(74) Representative: Hannke, Christian

(57) **Abstract**

The present invention relates to a system for modifying the tactile stimulus perceived by a user when touching a first object, wherein the system comprises a computing device and a vibration generator, wherein a data connection is established between the computing device and the vibration generator. The computing device is designed and configured to generate a output signal which correlates to a tactile stimulus of a second object, which is different with respect to the first object, wherein the vibration generator is designed and configured to generate a vibration signal based on the output signal, wherein the vibration signal can be perceived by a tactile receptor of the user.

Furthermore, the invention relates to a method for modifying the tactile stimulus perceived by a user when touching a first object.

## Description

The present invention relates to a system for modifying the tactile stimulus perceived by a user when touching a first object. Furthermore, the invention relates to a method for modifying the tactile stimulus perceived by a user when touching a first object.

In the past many systems have been developed, by which a user's sensory perception can be influenced, altered, or enhanced. Very common systems of this kind are amending the user's visual perception by providing a so-called (visual) augmented reality (AR). This technic allows to freely modify the appearance of the world around the user. Furthermore, headphones and in-ear speakers are known, which allow to modulate the sounds of the surrounding environment, e.g. by so-called noise cancelling.

Both amendments of the user's sensory perception are very important for applications using a virtual or augmented reality (VR or AR). Known applications of augmented or virtual reality include entertainment (particularly video games), education (such as medical, safety or military training) and business (such as virtual meetings).

However, the performance of these systems is limited because important stimuli such as the temperature, gustatory or olfactory senses cannot be triggered in accordance with the visually or acoustically altered reality. Similarly, the sense of touch has so far not been integrated in augmented reality.

There are known systems for creating various tactile material experiences. However, most of these require instrumenting the object one interacts with by adding sensing and actuation mechanisms. This limits the number of objects that can be augmented.

Thus, it is an object of the present invention to provide a system and a method for modifying the tactile stimulus perceived by a user when contacting an object without the need of modifying the contacted object.

This problem is solved by the subject matter of the independent claims.

A first solution to this problem is a system for modifying the tactile stimulus perceived by a user when touching a first object. This system comprises a computing device and a vibration generator, wherein a data connection is established between the computing device and the vibration generator. The computing device is designed and configured to generate a output signal which correlates to a tactile stimulus of a second object, which is different with respect to the first object. The vibration generator is designed and configured to generate a vibration signal based on the output signal, wherein the vibration signal can be perceived by a tactile receptor of the user. Thus, the tactile stimulus perceived by a user can be altered from the tactile stimulus of the first object to the tactile stimulus of a second object. Accordingly, when touching the first object, the user perceives this as if they were touching the second object. This modified tactile stimulus perceived by the user is caused by the applied vibration.

In a preferred embodiment of the system the vibration generator is directly or indirectly connected to a hand, preferably a finger of the user. Thus, the vibration generator is in direct contact with the user and independent from the object the user touches. Accordingly, it is not necessary to modify the first object as such, because the tactile stimulus perceived by the user can be amended independently from the object.

Preferably, the vibration generator is arranged on the dorsum of a hand and/or a backside of a finger. This arrangement allows the user to use his fingers normally and - in case the system is switched of, or no modified tactile stimulus is requested - sensing the first object as it is. Preferably the vibration generator is arranged on a fingernail. It has been found that a vibration signal provided on a fingernail can be felt on the palmar side of the fingertip, where the density of touch-sensitive nerve cells is particularly high.

In a preferred embodiment of the system, the vibration generator is part of a glove and/or a device which can be arranged at the dorsum of the hand and/or a backside of a finger. Especially a glove is easy to handle and can be put on quickly. However, gloves usually also cover the fingertips, which will result in an amended tactile stimulus, even when the system is switched of, or no amended tactile stimulus is needed. If exposed fingertips are required, a glove can be modified accordingly or another method of attaching the vibration generator can be used. Attaching the vibration generator to a fingernail has been found to be especially comfortable, since an (preferably removable) adhesive could be used. The adhesive strength of known adhesives on the fingernail is sufficiently high to ensure a secure fastening of the vibration generator on the fingernail.

Preferably the vibration generator is configured to emit a vibration of ≥ 50 Hz, preferably ≥ 60 Hz, preferably ≥ 70 Hz, more preferably ≥ 75 Hz, and most preferably ≥ 80 Hz and ≤ 500 Hz, preferably ≤ 400 Hz, preferably ≤ 450 Hz, preferably ≤ 400 Hz, more preferably ≤ 350 Hz, most preferably ≤ 300 Hz. A vibration in this range is especially suitable to modifying the tactile stimulus perceived by the user about at least 10 Shore A levels, preferably ≥ 20 Shore A levels, more preferably ≥ 25 Shore A levels, most preferably ≥ 30 Shore A levels.

Preferably, the system comprises a sensor device, which is designed and configured to detect contact between the tactile receptor of the user and the first object. By such a sensor, it can be detected whether the user (e.g. with their fingertip) contacts a first object. Preferably only in case a contact between the user and the first object is detected, a vibration is generated. This is advantageous since the user does not always perceive a vibratory signal, but only when the first object is contacted. Preferably, the sensor device comprises a force sensing system. The force sensing system can be arranged on the fingertip but preferably the sensor device does not interfere with the contact between the user's finger and the first object. Preferably, the sensor device comprises an optical component by which the contact between the user and the fist object can be detected. If a force sensor is used to detect any contact between the user (especially the user's finger) and the first object, the force sensor is preferably remote from the contact area. It has been found that a force sensor arranged on a side or at the back of a fingertip is suitable to detect contact of the respective finger and the first object because of the resulting deformation of the fingertip. Such an arrangement of the sensor device can be realized by integrating the sensor device in a glove.

Accordingly, in a preferred embodiment of the system, the sensor device is part of a glove and/or arranged on a backside of a finger, preferably on a fingernail.

Preferably, the vibration consists of a series of impulses or grains, which correlate in density to the amount of change in pressure. That is, a fixed or variable grain level and/or granularity is defined, which correlates to the number of grains the user encounters when changing pressure by a fixed amount. This grain level and/or granularity is preferably defined in grains per signal change. In this context, "signal change" should be understood as a change in the signal by a specified amount. Preferably, the signal change is a change in the applied force by a predefined amount. The amount can be positive (increased signal strength, e.g. increased force) or negative (reduced signal strength, e.g. reduced force). Accordingly, amending the signal strength by a large amount therefore results in the specified amount being exceeded several times in a short period of time.

Preferably, the vibration generator is part of a virtual reality simulation device. Thus, the generated vibration can further intensify the user's feeling of immersion in a virtual reality. More preferably, the virtual reality simulation device comprises goggles and/or a headset. Thus, the visual and/or acoustical displays that show the user a virtual reality are supplemented by a tactile display.

Virtual reality (VR) should be understood as a simulated experience that employs at least one display to give the user an immersive feel of a virtual world. The display can visually represent a virtual world and/or give the user an impression of a virtual environment in some other way. Often, the user's movements are detected, and the influence of this movement is mapped in the virtual world.

In the context of the present invention also augmented reality (AR) should be understood as being encompassed by the term "virtual reality", since augmented reality is an interactive experience that combines the real world and a virtual reality. Thus, the virtual reality not only contains virtual objects but also objects which are present in the real world. The content can span multiple sensory modalities, including visual, auditory, haptic, somatosensory and olfactory.

While Virtual Reality (VR) goggles usually completely isolate users from their environment, Augmented Reality (AR) glasses take a different approach. They are designed to be perceptually transparent. That is, they can provide access to virtual information without impeding the perception of the physical world around us. Accordingly, interaction between the user and the surrounding environment is possible.

In an advantageous embodiment of the system a perceived tactile stimulus of contacting the second object correlates to a shore A level which is ≥ 2, preferably ≥ 5, preferably ≥ 8, preferably ≥ 10, preferably ≥ 15, preferably ≥ 20, more preferably ≥ 25, most preferably 30 Shore A levels lower than a perceived tactile stimulus of contacting the first object. Thus, for example an automotive tire tread having a shore A value of about 70 is perceived by the user as having a reduced hardness and a shore A value of only about 55, which correlates to soft materials, e.g. a door seal.

Preferably the system allows amending the perceived tactile stimulus to a shore level which is ≤ 20 shore A levels, preferably ≤ 10 shore A levels, more preferably ≤ 5 shore A levels, most preferably ≤ 2 shore A levels away from the intended hardness of the second object.

The present invention is also directed to a method for modifying the tactile stimulus perceived by a user when touching a first object as described below. This method is preferably performed using a system as described above. Accordingly, all features disclosed with respect to the system should be considered as being disclosed as a feature of a component, on which at least one step of the method is performed. Likewise, each step described for the method should be understood as meaning that a component of the system is designed and set up to be used for this purpose.

A further solution to the above-mentioned problem is a method for modifying the tactile stimulus perceived by a user when touching a first object. The method comprises the steps of:
a) providing a computing device;
b) providing a vibration generator;
c) establishing a data connection between the computing device and the vibration generator;
d) calculating an output signal by the computing device, wherein the output signal correlates to a tactile stimulus of a second object, which is different with respect to the first object,
e) generating a vibration signal by the vibration generator, wherein the vibration signal is based on the output signal; and
f) applying the vibration signal to a surface where it can be perceived by a tactile receptor of the user.

Preferably, the method comprises the step of bringing the vibration generator in contact with a hand and/or a finger of the user. Since the density of touch-sensitive nerve cells is particularly high in the hand and especially in the fingertip, generated vibrations could be perceived in high resolution. Preferably the vibration generator is brought in contact with a dorsum of a hand and/or a backside of a finger, more preferably a fingernail. This allows direct contact of the hand's or finger's inner side with the object to be touched.

In a preferred variant of the method, the vibration signal comprises a wave of ≥ 20 Hz, preferably ≥ 30 Hz, preferably ≥ 40 Hz, more preferably ≥ 50 Hz, and most preferably ≥ 60 Hz and ≤ 1000 Hz, preferably ≤ 800 Hz, preferably ≤ 500 Hz, preferably ≤ 400 Hz, most preferably ≤ 300 Hz. These frequencies are preferred since in this range one of the four major types of mechanoreceptors, namely the Pacinian corpuscles is especially sensitive.

Preferably, the method comprises the step of detecting a contact between the user and the first object by means of a sensor device. The sensor can for example be an optical sensor. However, the sensor device preferably comprises a force sensitive resistor. A force applied to the force sensitive resistor as a result of the contact between the user and the first object can be easily detected. This is even possible if the force sensitive resistor is not arranged in the area where the contact occurs. Even deformations of the hand or finger caused by the contact between the user and the first object result in a detectable force. Preferably the sensor device is part of a glove and/or arranged on a backside of a finger, preferably on a fingernail.

In a preferred variant of the method, an optical sensor is used to detect contact force, for example by measuring changes in the blood volume of the finger contacting the first object.

In a preferred variant of the method, the calculation performed in step d) is based on a value, which is detected by the sensor device. The value preferably correlates to a force which is applied by the user to a surface of the first object. The force can be measured by a force sensitive resistor or being calculated from other values, i.e. muscle tension.

Preferably a further vibration signal is generated every time a measured value, preferably a measured value correlating to a force, exceeds a multiple of a previously defined difference value.

Preferably the rate at which vibration signals (or grains) are produced and/or emitted is proportional to the first derivative of the measured force signal. It has been found, that such a rate is considered to give a vibrotactile feedback which is considered to be very natural. Furthermore a rate can be easily calculated based on the first derivative.

Further advantages, objectives and features of the present invention will be described, by way of example only, in the following description with reference to the appended figures.

The figures show:
- Fig. 1: a schematic illustration of a vibration generator attached to a fingernail,
- Fig. 2: a schematic illustration of a system for modifying the tactile stimulus worn by a user,
- Fig. 3a) - c): a schematic illustrations of possible application areas,
- Fig. 4: a schematic illustration of preferred criteria for generating vibrotactile signals,
- Fig. 5a), 5b): an illustration of a test system (A) and recorded parameters (B),
- Fig. 6a) - 6e): test specimen of different geometries,
- Fig. 7: diagram showing a comparison of actual hardness of a test specimen and per-ceived hardness of this test specimen,
- Fig. 8a), 8b): illustrations of the effect of Shore A level on perceived softness (left) and the effect of grain number on perceived Softness (right).

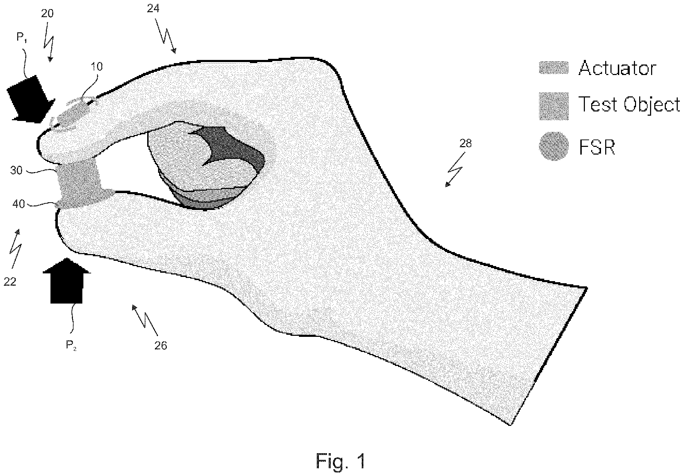

Fig. 1 shows a schematic illustration of a vibration generator 10 attached to a fingernail 20. By the vibrotactile signal generated by the vibration generator 10 the nerve cells in the fingertip 22 can be stimulated. The vibrotactile signal generated by the vibration generator 10 contributes to an entire vibrotactile stimulus, which also includes the tactile stimuli caused by contact between the fingertip and the object 30. Thus, the entire received stimulus is different with respect to the tactile stimuli caused by contacting the object 30. The depending on the additional vibrotactile signal generated by the vibration generator 10 the entire received stimulus can be amended.

In a preferred embodiment, the additional vibrotactile signal generated by the vibration generator 10 is generated depending on a difference of a force (f) applied to the object 30. In the illustrated experimental setup, the force (f) applied by the forefinger 24 and thumb 26 of a user's hand 28 to the object 30 along the directions indicated by the arrows P1 and P2 was detected by a force sensitive resistor 40. It has been found that the location of the force sensitive resistor 40 can be changed. It can for example also be arranged on a side of the forefinger 24 and/or thumb 26 and detect a deformation of the respective finger caused by the applied pressure. Other systems can also be used, e.g. systems which optically measure the acceleration of a fingertip 20 towards the object 30.

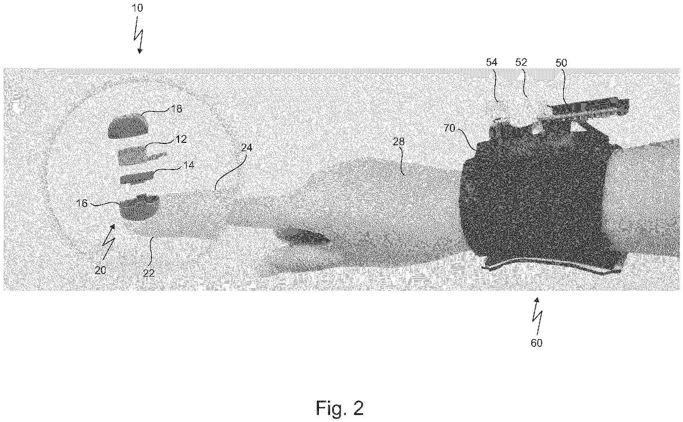

Fig. 2 is a schematic illustration of a system 1 for modifying the tactile stimulus worn by a user. The system 1 comprises a computing device 50 and the vibration generator 10. Both are interconnected by a data connection, which can be wireless. In the experimental setup, the vibration generator 10 was connected with the computing device 50 by a 5-lead cable (not shown), which is integrated into a custom-made, expandable neoprene wristband (60).

In fig. 2 the vibration generator 10 is shown in an exploded view. Thus, the actuator 12 and a (preferably optical) pressure sensor 14 can be seen between a first part 16 and second part 18 of a vibration generator housing. The vibration generator 10 includes a vibrotactile actuator 12 (LRA, Vybronics VLV101040A13) on top of the pressure sensor (unit) 14. This vibrotactile actuator 12 delivers vibrations along the Z-axis, with a peak frequency response at 170Hz. Both components are integrated in the housing, which in the illustrated example has a dimension of 180×160×150mm. at least the first part 16, which contacts the finger and/or fingernail when used, was fabricated using a flexible material (e.g. Formlabs Flexible V2 resin14). Thus, the first part 16 can accommodate various finger sizes and fingernail shapes while minimizing ambient light's influence on sensor readings. To ensure a secure fit and mitigate potential sensor noise caused by unintended movement, the first part 16 was adhered to the fingernail 20 using make-up adhesive (Kryolian Mastix professional grade spirit gum15).

Preferably the system combines sensing and vibrotactile actuation within a convenient wearable form factor. Thus, the computing device 50 and a respective power supply is preferably arranged in a bracelet 60 that can be worn by the user. This bracelet houses the components for generating the compliance illusion. It can include a power supply 70 to make the system 1 portable. As computing device 50 a Microcontroller Unit (MCU) (in this case a Teensy 4.116) was used. It reads analog signals from the pressure sensor (unit) 14 of the vibration generator 10 and subsequently generates grains, short sinusoidal impulses, in accordance with the user's applied pressure. This digital signal is then converted into an analog signal by a DAC (in this case a PT821117) (reference sign 52), which is further amplified (through an Adafruit PAM8302 2.5W mono amplifier18) (reference sign 54) before being delivered to the actuator 12 within the housing 16, 18. The DAC and the amplifier are both included in the bracelet 60.

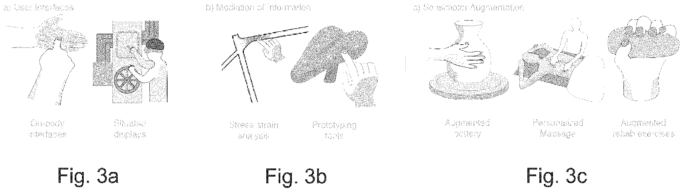

Fig. 3a) - 3c) are schematic illustrations of possible application areas.

As illustrated in fig. 3a) the system could be used for user interfaces (Ul) augmenting on body, or situated displays with tactile interactivity. It has been found that the system can make rigid objects feel more interactive. This makes a natural application space of the system for creating responsive Uls in tactile AR. Only as exemplary applications systems that project interfaces on the body (left) or situated displays (right) are illustrated. For both applications the system could be augmented to provide satisfying tactile feedback to user input. These temporary displays often include a corresponding Ul. They can be dynamically deployed. Using the system described above, such displays can similarly be augmented with a satisfying tactile component to the interaction by simulating force compliance-curves, compression, or clicks of tactile UI elements. In an environment where these systems are used, it might also make sense to use these systems to augment digital technologies such as smart-watches or tablets.

As illustrated in fig. 3b) the system could be used for mediation of information. In this context the system can serve as a tool for users to access invisible information. Such information can be provided in an embodied or hermeneutic, symbolic manner.

An example of embodied mediation is illustrated on the right of fig. 3b. For embodied mediation, information is presented in a way that a user understand pre-reflectively. This is the default way of interacting with the system. Designers might wish to 3D print the rigid shape of an object, and then use the system to explore different levels of softness. For example, one designer might create a 3D shape of a bicycle saddle and send it to multiple colleagues. Each colleague could then design the softness of the saddle. Different softness profiles could be shared among all designers and tested on the same rigid 3D print. Here, the information that is mediated is the compliance itself.

An Example of a hermeneutic or symbolic approach is illustrated on the left of fig 3b). The compliance might be used as a symbol representing something else. For example, the same team of bicycle designers might also prototype different frame geometries and run a stress-strain analysis on these. The designers can now tactually explore the result of these models on the prototype frames with the system, for example, by making the fragile places where strain is the highest feel softer than the places that are not under strain. In this scenario, the compliance is a symbol, a placeholder which the users interpret to represent other information.

In fig. 3b) exemplary applications for providing information about user actions in the real world are shown. The system could be used as sensory augmentation - or rather, as a so-called sensorimotor augmentation - providing users with heightened sensitivity to fine motor activity. This can be used to support tasks where precise pressure levels are important, for example, it might support novices in learning how to use a potter's wheel (fig. 3c, left). The un-occluded fingertip still allows the novice to feel the rich material properties of the clay, while at the same time the feedback from the system helps them to keep the pressure at the desired level.

This feedback could also be collaboratively shaped. For example (shown in the middle of fig. 3c), a person offering professional massages might let their customer set maximum pressure levels for their comfort. The massage therapist could then feel, in real-time, the pressure level the customer wants, while at the same time having their fingers un-occluded for skin-to-skin interaction. As the fingers are un-occluded, the person offering the massage can also use oils and creams without them interfering with the system.

The system as sensorimotor augmentation could be used as a rehabilitation tool (fig 3c, right). For example, stroke patients often require extensive rehabilitation exercises after paralysis, often using physical objects like balls to regain hand mobility. This process can be frustrating, especially since initial progress is slow. The system can enhance this rehabilitation process by enabling a certain category and grade of stroke patients to feel their movements better. The added sensory feedback to even the smallest movement can provide patients with encouragement that they are actually making progress.

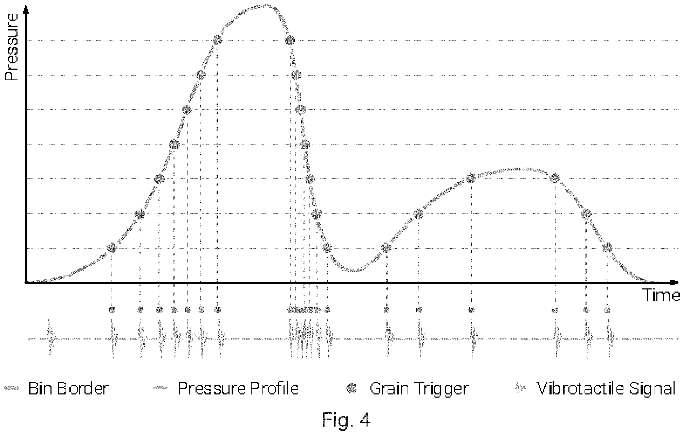

Fig. 4 is a schematic illustration of preferred criteria for generating vibrotactile signals. The graphics visualizes how an action-coupled signal could be generated. In this example pressure is sampled over time. As the signal (solid line) crosses fixed pressure thresholds (horizontal dotted lines) a grain trigger (dots) is added to the haptic drive signal (bottom).

The grain-based compliance illusion can be used to make rigid objects feel compliant. This effect is achieved by measuring the applied force while users apply pressure and playing short vibration pulses (grains) when crossing predefined force values. This results in pulse frequencies that always correlate with the change in pressure. If the user keeps the pressure steady, they will feel no pulses. If the user changes the pressure, they feel rapid pulses. It is referred to signals with this type of synchronization as coupled to human action. An important aspect of this illusion is that, when the coupling is tight enough, the vibrotactile signal is no longer experienced as vibration, but purely as an effect of the complying material.

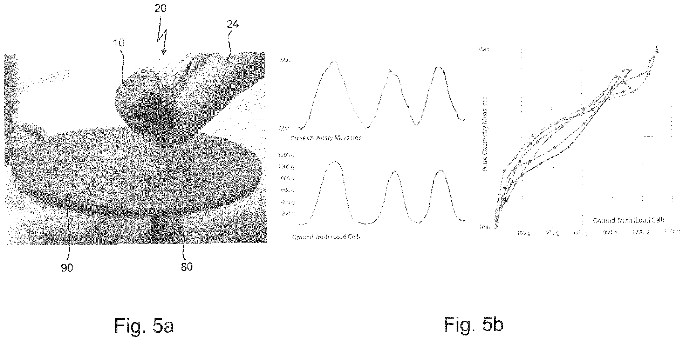

Fig. 5a) and 5b) are an illustration of a test system (a) and recorded parameters (b). It was found to be advantageous, to allow users to maintain a connection with the real-world tactile properties while benefiting from augmenting their perception with motion-couple vibrotactile feedback. To allow this, an optical system to measure finger pressure was used. Miniaturized LEDs and photo-detectors commercially available today were found to be well-suited for integration into a nail-based solution. Among various documented optical systems in the literature, a system having a LED emitting light in the near infrared range, and a respective photodetector was used to detect changes caused by applied finger pressure. A system capable of irradiating and measuring infrared light through the finger, with pulse oximetry was found to be most suitable.

The design approach of photoplethysmograph (PPG) fingernail sensors was considered as most beneficial because of their ability to be easily attached and removed from the nail, making them suitable in a non-permanent wearable scenario. PPG offers a simple and cost-effective way to monitor changes in blood volume within the microvascular tissue bed. In the first experiments a MAXREFDES117 sensing module board, which is centered around a MAX30102 Pulse Oximeter and Heart-Rate sensor was used. This sensor was equipped with integrated red (660 nm) and IR (880 nm) LEDs and a medium band photodetector (600-900 nm). Utilized in a reflectance PPG configuration, it offers precise readings of blood volume fluctuations in the fingernail bed peripheral circulation when pressure is applied. Moreover, the sensor incorporates an ambient light cancellation system to reduce the impact of environmental sensing artifacts.

The sensing board is located in the lower layer of a 3D-printed housing 16, 18 that is attached to the nail 20 using make-up adhesive. The sensor 10 was placed as close to the middle of the nail 20 as possible and be tightly adhered to prevent accidental displacements on the Z axis which can lead to undesired sensing artifacts. The adhesive can be easily removed. The sensor's analog-to-digital converter (ADC) configuration was adjusted to transmit an 18-bit data value via I2C to a Teensy 4.1 Micro Controller Unit (MCU, not shown).

The performance of the proposed finger pressure sensor was assessed by comparing it with a TAL220 20 kg load cell12 (reference sign 80), as a baseline. To this purpose, a test bed for assessing the capabilities of the finger pressure sensor was set up as shown in Figure 5a. This setup comprises a flat plastic surface 90, along with a load cell 80 situated beneath the surface 90 to capture force measurements, serving as baseline. This testing setup allowed direct comparison between the optical sensor 10 and the load cell 80 under various levels of pressure applied to the surface 90.

Participants with diverse nail colors were instructed to press their finger equipped with the optical sensor onto the test surface 90. The data from the optical sensor and the load cell were recorded simultaneously. The pressure data received from the optical sensor were compared with the load cell's force data to evaluate the performance of the optical sensor. As illustrated in fig. 5b) the optical sensor performed well.

It has been found that the correlation between the data measured based on the optical sensor and the load cell could even be improved if side effects like finger bending and heartbeat are considered. Side effects caused by finger bending could be corrected using a finger-movement machine learning detection model. The side effects caused by the heartbeat could be effectively filtered by employing predictive filters such as Adaptive notch filters (ANF) that have been used to extract heart rate from raw PPG signals. Moreover, user-specific calibration of minimum and maximum values have been performed because individuals have varying blood volume and light translucence in the fingernail area.

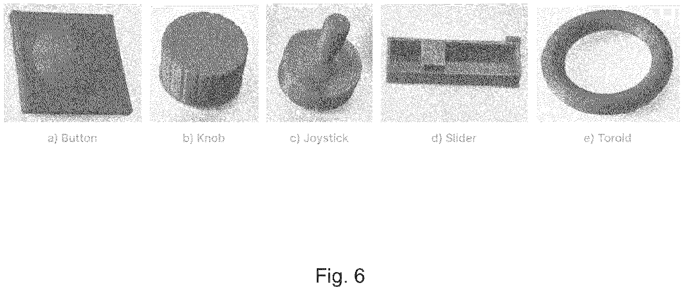

Fig. 6a) - 6e) show examples of test specimen of different geometries, which have been used for further studies with participants. During the study, participants equipped with a system according to the present invention interacted with a variety of tangible objects, such as 3D-printed rigid buttons (fig. 6a), joysticks (fig.6c), sliders (fig. 6d), toroids (fig 6e), and knobs (fig. 6b). Upon augmentation with the haptic feedback, each object impaired distinct tactile experiences.

Participants were seated in a chair in front of a desk. Both hands were positioned behind a black curtain, which ensured that no visual cues were available while exploring the objects. For each of the 3D printed shapes, the participants reported that while pressing against a rigid object, the system provides an experience of virtual compliance. The geometries of the different tangibles also provided participants with additional contextual information, which changed their perception of compliance to be contextually meaningful within the interaction. For example, participants identified differences in the intensity and nature of feedback between knobs and buttons. Some noted that buttons produced clear on/off sensations, while knobs offered varying resistance and granularity even though the same feedback was delivered. This distinction in feedback contributed to the perception of different control functionalities.

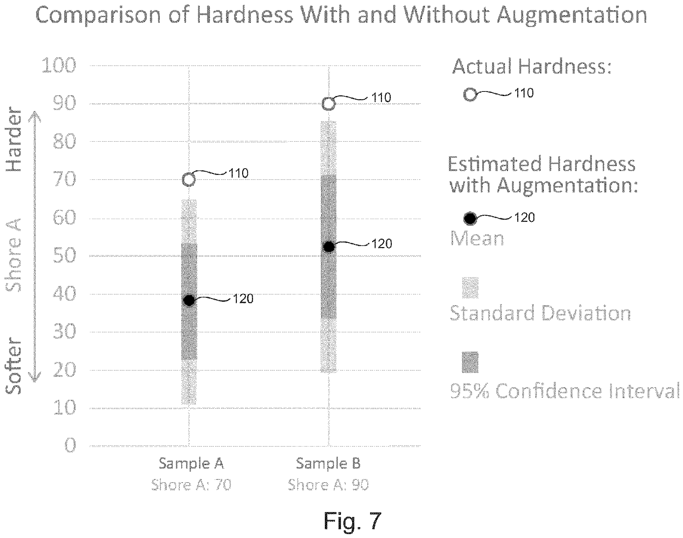

Fig. 7 is a diagram showing a comparison of actual hardness of a test specimen and perceived hardness of this test specimen. For all participants it was ensured that no visual cues were available while exploring the test specimen. The test specimen were 3D-printed cuboids (6x6x7 mm) of Shore-A levels 10A, 20A, 30A, 40A, 50A, 60A, 70A, 80A, and 90A. For the illustration in fig. 7 two relatively hard objects were selected, having a Shore-A value of 70A and 90A. Relatively hard objects had been chosen since these would allow for the largest reduction in hardness.

For the test, participants were equipped with a system according to the invention. A 1-up, 1-down adaptive staircase design was used to determine the level of softness induced by the compliance illusion on rigid objects. In each trial of the staircase, participants interacted simultaneously with two samples: (1) a test object touched by one of their index fingers, with an actuator attached to the fingernail, and (2) a reference object touched by the index finger of the opposite hand, without an actuator. Participants were then asked to indicate whether the test object felt softer than the reference object. In the staircase design, if participants responded with "yes" (indicating that the test object felt softer), the hardness of the subsequent reference stimulus was decreased by 1 (resulting in a softer reference object being presented). Conversely, if participants responded with "no" (indicating that the test object did not feel softer), the hardness of the next reference stimulus was increased by 1 (resulting in a harder reference object being presented), following the conventions of traditional staircase study design. Each participant completed four staircases. Each staircase was followed until five reversals were reached. The final discrimination value was obtained by calculating the average of the last three reversals. For the tactile augmentation system, a granularity of 40 with 200 Hz tactile grains lasting 5ms was selected.

In fig. 7 the actual hardness of test specimen is visualized by the circles 110. The graph on the left shows the results of a test with a test specimen with a Shore A value of 70, whereas the right one shows results of a test with a test specimen with a Shore A value of 90. The red dots 120 indicate the average perceived hardness across all participants. For moth scenarios the average perceived hardness is more than 30 Shore A values lower than the actual Shore A value of the test specimens. The bars illustrate the respective standard deviation and the 95% confidence interval. These results highlight that the compliance illusion does indeed work, when the back of the finger is stimulated.

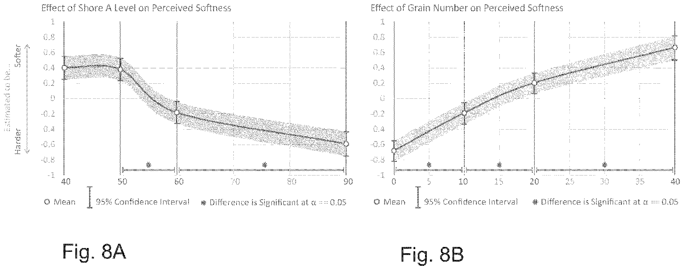

Fig. 8a shows an illustration of the effect of Shore A level on the perceived softness and fig. 8b the effect of the grain number on the perceived softness. For the experiments underlaying these illustrations, participants were seated in a chair in front of a desk with a display and keyboard. Their dominant hand was positioned behind a black curtain, which ensured that no visual cues were available while exploring the test specimen. Participants were asked to rate each combination of physical sample and tactile feedback, by entering a number in a GUI. During the experiment, test specimen with different hardness were crossed with four levels of tactile stimulation. The test specimen had Shore levels of 40A, 50A, 60A, and 90A.

The participants were equipped with a system according to the present invention and vibrotactile stimuli were generated. The number of grains selected for this experiment were 0 (i.e., no augmentation), 10, 20, and 40 grains. The vibrotactile stimuli were grain-based compliance illusions implemented using haptic servos. The pressure range was divided into discrete sections (bins), where a grain is played on each crossing of a bin (as also illustrated in fig. 4). The number of bins over the entire range defines the granularity of the stimulus. A single grain is a short vibrotactile pulse that is characterized by vibration properties such as frequency, amplitude (intensity), and duration. Each grain was a 5 ms long 200 Hz sinewave.

In the standardized scale used in fig. 8a and 8b, an estimate of one indicates that this estimate is one standard deviation above the average estimate. This highlights differences between conditions, while removing individual differences between participants. The response curves depicting virtual and physical compliance are shown. It was found that, with increasing Shore-A level, participants rated the samples less compliant (fig. 8a).

Notably, the response curve for virtual compliance shows a clear trend: as the number of grains in the vibrotactile rendering increases, the perceived softness of the explored object also increases (fig. 8b).

The applicant reserves his right to claim all features disclosed in the application document as being an essential feature of the invention, as long as they are new, individually or in combination, in view of the prior art. Furthermore, it is noted that in the figures features are described, which can be advantageous individually. Someone skilled in the art will directly recognize that a specific feature being disclosed in a figure can be advantageous also without the adoption of further features from this figure. Furthermore, someone skilled in the art will recognize that advantages can evolve from a combination of diverse features being disclosed in one or various figures.

### List of reference symbols

- 1: System
- 10: vibration generator, actuator unit, sensor unit
- 12: actuator
- 14: (optical) pressure sensor
- 16, 18: first part and second part of a housing
- 20: fingernail
- 22: fingertip
- 24: finger
- 26: thumb
- 28: hand
- 30: object
- 40: force sensitive resistor
- 50: computing device, Microcontroller Unit
- 52: digital analog converter, DAC
- 54: amplifier
- 56: Amplifier, Visaton 2.2LN amplifier
- 60: bracelet
- 70: power supply
- 80: load cell
- 90: test surface, plastic surface
- 110: circle, actual hardness
- 120: dot, estimated hardness
- P1, P2: Arrows

## Claims

1. A system (1) for modifying the tactile stimulus perceived by a user when touching a first object (30), wherein the system (1) comprises a computing device (50) and a vibration generator (10, 12), wherein a data connection is established between the computing device (50) and the vibration generator (10)
**characterized in that**
the computing device (50) is designed and configured to generate a output signal which correlates to a tactile stimulus of a second object, which is different with respect to the first object (30), wherein the vibration generator (10, 12) is designed and configured to generate a vibration signal based on the output signal, wherein the vibration signal can be perceived by a tactile receptor of the user.

2. A system (1) according to claim 1, **characterized in that** the vibration generator (10, 12) is directly or indirectly connected to a hand (28), preferably a finger (24) of the user.

3. A system (1) according to claim 1 or 2, **characterized in that** the vibration generator (10, 12) is arranged on the dorsum of a hand (28) and/or a backside of a finger (24), preferably on a fingernail (20).

4. A system (1) according to any of claims 1 to 3, **characterized in that** the vibration generator (12) is part of a glove and/or a device (10) which can be arranged at the dorsum of the hand (28) and/or a backside of a finger (24).

5. A system (1) according to any of claims 1 to 4, **characterized in that** the vibration generator (12) is configured to emit a vibration of ≥ 50 Hz, preferably ≥ 60 Hz, preferably ≥ 70 Hz, more preferably ≥ 75 Hz, and most preferably ≥ 80 Hz and ≤ 500 Hz, preferably ≤ 400 Hz, preferably ≤ 450 Hz, preferably ≤ 400 Hz, more preferably ≤ 350 Hz, most preferably ≤ 300 Hz.

6. A system (19 according to any of claims 1 to 5, **characterized by** a sensor device (14), which is designed and configured to detect contact between the tactile receptor of the user and the first object (30), wherein the sensor device (14, 40) preferably comprises a force sensing system (40) and/or an optical sensor (14), wherein preferably the sensor device does not interfere with the contact between the users finger (24) and the first object (30).

7. A system (1) according to claim 6, **characterized in that** the sensor device (14) is preferably part of a glove and/or arranged on a backside of a finger (24), preferably on a fingernail (20).

8. A system (1) according to any of claims 1 to 7, **characterized in that** the vibration generator (10,12) is part of a virtual reality simulation device, wherein the virtual reality simulation device preferably comprises goggles and/or a headset.

9. A system (1) according to any of claims 1 to 8, **characterized in that** a perceived tactile stimulus of contacting the second object correlates to a shore A level which is ≥ 2, preferably ≥ 5, preferably ≥ 8, preferably ≥ 10, preferably ≥ 15, preferably ≥ 20, more preferably ≥ 25, most preferably 30 shore A levels lower than a perceived tactile stimulus of contacting the first object (30).

10. Method for modifying the tactile stimulus perceived by a user when touching a first object (30), the method comprising the steps:
a) providing a computing device (50);
b) providing a vibration generator (10, 12);
c) establishing a data connection between the computing device (50) and the vibration generator (10, 12);
**characterized by**
d) calculating an output signal by the computing device (50), wherein the output signal correlates to a tactile stimulus of a second object, which is different with respect to the first object (30);
e) generating a vibration signal by the vibration generator (12), wherein the vibration signal is based on the output signal; and
f) applying the vibration signal to a surface (20) where it can be perceived by a tactile receptor of the user.

11. Method according to claim 10, comprising the step of bringing the vibration generator (10, 12) in contact with a hand (28) and/or a finger (24) of the user, preferably a dorsum of a hand (28) and/or a backside of a finger (24), more preferably a fingernail (20).

12. Method according to claim 10 or claim 11, wherein the vibration signal comprises a wave of ≥ 20 Hz, preferably ≥ 30 Hz, preferably ≥ 40 Hz, more preferably ≥ 50 Hz, and most preferably ≥ 60 Hz and ≤ 1000 Hz, preferably ≤ 800 Hz, preferably ≤ 500 Hz, preferably ≤ 400 Hz, more preferably ≤ 350 Hz, most preferably ≤ 300 Hz.

13. Method according to any of claims 10 to 12, comprising the step of detecting a contact between the user and the first object (30) by means of a sensor device (14), wherein the sensor device (14, 40) preferably comprises a force sensitive resistor (40) and/or an optical sensor (14), which is preferably part of a glove and/or arranged on a backside of a finger (24), preferably on a fingernail (20).

14. Method according to any of claim 13, wherein the calculation performed in step d) is based on a value, which is detected by the sensor device (14, 40), wherein the value preferably correlates to a force which is applied by the user to a surface of the first object (30).

15. Method according to claim 13 or 14, wherein a further vibration signal (grain) is generated every time a measured value, preferably a measured value correlating to a force, exceeds a multiple of a previously defined difference value.
